# EUROPEAN PATENT SPECIFICATION

(11) **EP 2 886 531 B1**
(45) Date of publication and mention of the grant of the patent: **08.06.2016**
(21) Application number: 14003926.4
(22) Date of filing: 21.11.2014
(51) Int. Cl.: C07C 271/22, C07C 229/24, C07K 1/06

(54) **Aspartic acid derivatives**
Asparaginsäurederivate
Dérivés d'acide aspartique

(30) Priority: 19.12.2013 EP 13005920
(43) Date of publication of application: 24.06.2015
(73) Proprietor: Merck Patent GmbH, 64293 Darmstadt (DE)
(72) Inventor: Behrendt, Raymond, 78224 Singen (DE); Willibald, Julian, 81543 Muenchen (DE); White, Peter, Southwell, NG25 0HQ (GB)

(56) References cited:
- WO-A1-95/26975
- CN-A- 102 260 327
- KARLSTROM A ET AL: "A New Protecting Group For Aspartic Acid That Minimizes Piperidine-Catalyzed Aspartimide Formation In Fmoc Solid Phase Peptide Synthesis", TETRAHEDRON LETTERS, PERGAMON, GB, vol. 37, no. 24, 10 June 1996 (1996-06-10) , pages 4243-4246, XP004029127, ISSN: 0040-4039, DOI: 10.1016/0040-4039(96)00807-6
- M. MERGLER ET AL: "The aspartimide problem in Fmoc-based SPPS. Part III", JOURNAL OF PEPTIDE SCIENCE, vol. 11, no. 10, 1 October 2005 (2005-10-01), pages 650-657, XP055181875, ISSN: 1075-2617, DOI: 10.1002/psc.668
- RAMON SUBIRS-FUNOSAS ET AL: "Aspartimide formation in peptide chemistry: occurrence, prevention strategies and the role of-hydroxylamines", TETRAHEDRON, vol. 67, no. 45, 2011, pages 8595-8606, XP028308691, ISSN: 0040-4020, DOI: 10.1016/J.TET.2011.08.046 [retrieved on 2011-08-23]

## Description

### Field of the Invention

The present invention is directed to derivatives of aspartic acid with a new type of side-chain protecting group. The present invention is further directed to methods of their preparation and their use in Fmoc based solid phase peptide synthesis as well as the Z/tBu based solution synthesis of peptides. The use of the aspartic acid derivatives according to the present invention leads to the minimization of base catalyzed aspartimide formation during peptide synthesis and supports the prevention of aspartimide derived impurities.

### Background of the Invention

Peptides can be manufactured by solid-phase synthesis. One widely used approach of peptide manufacturing is the Fmoc/tBu based solid phase peptide synthesis (Fmoc SPPS) which involves the use of 9-Fluorenylmethyloxycarbonyl (Fmoc) protection for the alpha-amino acid building blocks. The side-chain functionalities in trifunctional amino acids are herein protected by acid labile groups like *tert*-butyl esters leaving only the C-terminus open for specific stepwise peptide chain elongation. To assemble the peptide from the C- to the N-terminus the first C-terminal amino acid is immobilized on a solid support like a crosslinked polystyrene resin. An automatable cycle of Fmoc deprotection and peptide bond formation facilitates the peptide assembly. Complete conversions in the amide bond formation are ensured by an excess of the Fmoc amino acid and in-situ coupling reagent (see Sheppard. R.C., J. Peptide Sci., 9: 545-552 (2003)).

Besides, peptides can be manufactured via convergent synthesis. Peptide fragments mostly of six to ten amino acid length are assembled by Fmoc SPPS or alternatively in solution where the N-terminal benzyloxycarbonyl protection (Z) is predominantly used.

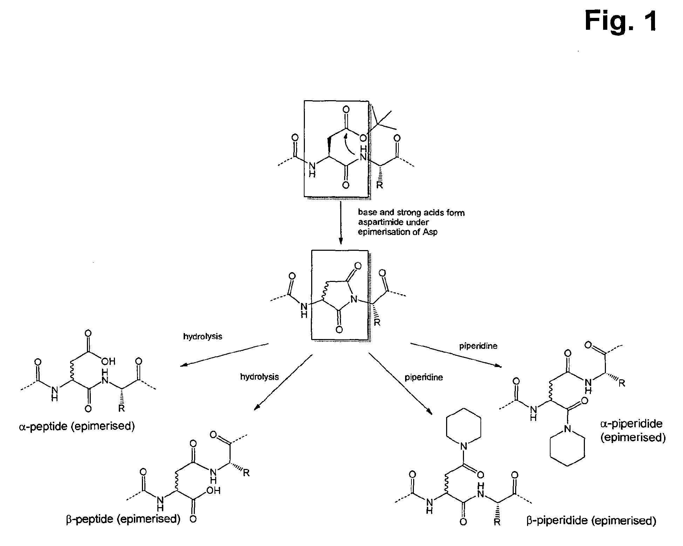

Among all side reactions in the Fmoc SPPS as well as in the Z based solution synthesis the appearance of aspartimide is one of the most severe issues (see Subiros-Funosas R. et al., Tetrahedron, 67: 8595-8606, (2011)). In susceptible sequences it occurs in all preceding Fmoc deprotection steps and amide bond formations using tertiary amine catalysis after the introduction of aspartic acid. Thereby it leads to a significant accumulation of related by-products. The aspartimide is generated by the base catalyzed cyclization of the side-chain carboxylic group with the C-terminal amide nitrogen. Ring opening of the succinimide moiety is then accompanied by epimerization and beta-isomerization forming eight by-products either through its hydrolysis or piperidine addition (Figure 1). All these by-products are modifications of the peptide core and are present in the desired peptide as well as in any deletion or truncated sequences containing the aspartic acid side-chain. Thereby a complex mixture of peptidic impurities is formed which emposes difficulties to separation of the desired compound. In solution this side reaction occurs merely during amide bond formation using tertiary amine catalysis (see Martinez, J.; Bodanszky, M., Int. J. Pept. Protein Res., 12:277-283 (1978)).

Sequences particularly susceptible to aspartimide formation are -Asp-Gly-, - Asp-Asp-, -Asp-Asn-, -Asp-Arg- and -Asp-Cys- (see Mergler M., et al., J. Peptide Sci., 9: 518-526 (2003)). While for any other sequence the standard Fmoc-Asp(OtBu)-OH derivative may be sufficient, the best currently commercially available derivative Fmoc-Asp(OMpe)-OH (see Karlström, A. et al., Tetrahedron Letters, 37: 4243-4246 (1996)) is still not sufficiently protecting against aspartimide formation in the above mentioned sequences.

The application of microwave heating to SPPS is particularly advantageous for long or aggregation prone peptides as the acceleration of coupling and deprotection reaction leads to shorter cycle times, higher repetitive yields, and ultimately purer peptides (see Pedersen L., et al., Chem. Soc. Rev., 41: 1826-1844 (2012)). However, the use of microwave energy also leads to increased levels of typical SPPS by-products including aspartimide formation which can only be limited by routine use of optimized methods (see Palasek S. A., et al., J. Peptide Sci., 13, 143-148 (2007)).

WO 95/26975 discloses aliphatic branched alkyl groups as constituents of protective groups for side chains of amino acids. Basis of this approach is the generation of sterically demanding, bulky protective groups to avoid side reactions like aspartimide formation. But these protective groups never gained much interest as due to the sterically demanding group also the reactivity of the protected amino acids in peptide synthesis is reduced. Also Mergler M., et al., J. Peptide Sci., 9: 518-526 (2003) discuss the use of bulky alkyl groups for side chain protection of aspartic acid but conclude that a total suppression of the aspartimide formation cannot be achieved by simply increasing the bulk of the side chain protection.

One example of a therapeutically important peptide family suffering from significant levels of aspartimide formation is glucagon-like peptide 2 (GLP-2) and its related analogs (WO 2012/028602). These peptides are suggested for the treatment of Crohn's disease osteoporosis and as adjuvant therapy in chemotherapy. The most prominent example is Revestive® (teduglutide) which is used for the treatment of the short bowel syndrome. GLP-2 analogs like Revestive® are currently manufactured recombinantly and an effective synthetic production method would be an interesting alternative to lower the production costs and to omit animal-derived starting materials as well as to reduce the endotoxine burden. However, these peptides contain an Asp-Gly as well as an Asp-Asn sequence which reduces their synthetical accessibility due to aspartimide derived by-products (see WO 2012/028602).

Clearly, the aspartimide issue still imposes drawbacks to the peptide synthesis and there remains a need in the art for a versatile and efficient protecting group of the aspartic acid side-chain preventing this side reaction and it gives a legitimate cause of concern that important peptide therapeutics encounter severe difficulties in their synthetic manufacturing. It has been found that aspartic acid side chain protection groups having a tertiary O-linked carbon atom with only linear aliphatic substituents can be efficiently used for peptide synthesis. Surprisingly, this type of protecting group combines effective hindrance of the side reaction with high reactivity of the protected aspartic acid in peptide synthesis.

### Summary of the Invention

The present invention is therefore directed to compounds of formula la and/or lb 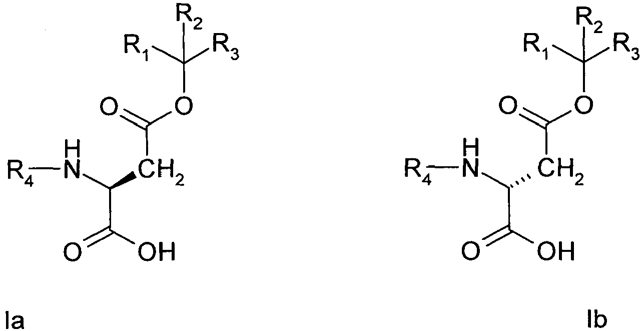 wherein
R₁, R₂, R₃ represent, independently of each other, a linear hydrocarbon chain of two to six carbon atoms length, e.g. -CH₂-CH₃, -CH₂-CH₂-CH₃, -CH₂-CH₂-CH₂-CH₃, -CH₂-CH₂-CH₂-CH₂-CH₃, -CH₂-CH₂-CH₂-CH₂-CH₂-CH₃.
R₄ represents a benzyloxycarbonyl or a 9-fluorenyl methoxycarbonyl group or H.

Formula II shows the benzyloxycarbonyl or the 9-fluorenyl methoxycarbonyl group.

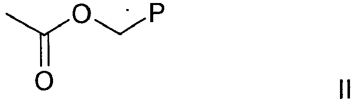

In formula II P represents a phenyl- or 9-fluorenyl group.

The two formula Iia and Ib represent the D and the L (R and S) enantiomers of aspartic acid. The compound can be either the pure D enantiomer or the pure L enantiomer or any mixture of the D and L enantiomers. Preferred is the L enantiomer.

In a preferred embodiment, R₁, R₂, R₃ all have the same number of carbon atoms.

In another preferred embodiment, R₁, R₂, R₃ are -CH₂-CH₃ or -CH₂-CH₂-CH₃.

The present invention is further directed to a method of manufacturing the compounds of formula la and/or Ib by
a) Providing an aspartic acid with hydrogenizable protecting groups at the N and C terminus
b) Esterification of the side chain carboxylic acid with the tertiary alcohol according to formula III

   HO-CR₁R₂R₃ formula III

   whereby R₁, R₂, R₃ represent, independently of each other, a linear hydrocarbon chain of two to six carbon atoms length, e.g. -CH₂-CH₃,-CH₂-CH₂-CH₃, -CH₂-CH₂-CH₂-CH₃, -CH₂-CH₂-CH₂-CH₂-CH₃, -CH₂-CH₂-CH₂-CH₂-CH₂-CH₃
c) Hydrogenolysis of the hydrogenizable protecting groups at the N and C terminus
d) Introduction of a fluorenylmethoxycarbonyl or a benzyloxycarbonyl group at the N-terminus. In a preferred embodiment, the esterification of step b) is performed with a carbodiimide preferentially dicyclohexylcarbodiimide.

The present invention is further directed to a method for peptide synthesis by
a) Providing an amino acid or a peptide bound to a solid phase via its C-terminus
b) Reacting the solid phase bound amino acid or peptide of step a) with a compound of formula la and/or formula Ib whereby the compound of formula Ia and/or Ib is attached to the N-terminus
c) Cleavage of the Fmoc group by alkaline treatment

In a preferred embodiment, the alkaline treatment of step c) is performed with one or more secondary amines, preferrably piperidine, 1,8-diazabicyclo[5.4.0]undec-7-en (DBU), pyrrolidine and/or diethylamine.

In a very preferred embodiment, the alkaline treatment of step c) is performed with a mixture comprising one or more secondary amines and an organic acid or preferably an acidic alcohol, e.g. ethyl cyanoglyoxylate-2-oxime. The concentration of the organic acid or the acidic alcohol should be chosen such that it is sub-stoichiometric as compared to the secondary amines.

In another preferred embodiment, the reaction in step b) is performed in the presence of HBTU, TBTU, HATU, TATU, HCTU, TCTU and/or PyBOP.

### Figures

Figure 1. Mechanism of the formation of the aspartimide derived by-products.
Figures 2-7 show HPLC and UPLC traces related to the examples.

### Definitions

An organic acid according to the present invention is an organic compound with acidic properties. The most common organic acids are the carboxylic acids, whose acidity is associated with their carboxyl group-COOH. Sulfonic acids, containing the group-SO₂OH, are relatively stronger acids. Alcohols, with -OH, can act as acids but they are usually very weak. The relative stability of the conjugate base of the acid determines its acidity. Other groups can also confer acidity, usually weakly: the thiol group-SH, the enol group, and the phenol group. Preferred organic acids according to the present invention are acidic alcohols.

An acidic alcohol according to the present invention is an alcohol with an OH group that can act acidic. Examples are (Z)-ethyl cyanoglyoxylate-2-oxime (Oxyma Pure), N-hydroxybenzotriazole and phenols.

### Detailed Description of the Invention

The present invention provides derivatives of aspartic acid for the peptide synthesis either in solution or on a solid support. The side-chain protection comprises an ester bonded tertiary alcohol having a tertiary C-atom with straight hydrocarbon chains with two to six carbon atoms. The side chain protected aspartic acid derivatives according to the present invention are shown in formula la and Ib. 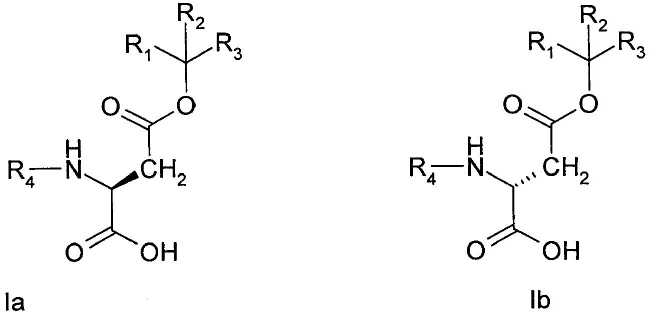 wherein
R₁,R₂,R₃ represent, independently of each other, a linear hydrocarbon chain of two to six carbon atoms length, e.g. -CH₂-CH₃, -CH₂-CH₂-CH₃, -CH₂-CH₂-CH₂-CH₃, -CH₂-CH₂-CH₂-CH₂-CH₃, -CH₂-CH₂-CH₂-CH₂-CH₂-CH₃
R₄ represents a benzyloxycarbonyl or a 9-fluorenylmethoxycarbonyl group or H.

The two formula la and Ib represent the D and the L (R and S) enantiomers of aspartic acid. The enantiomers can be used as pure D or L compound or as a mixture.

It has been found that it is favourable if R₁, R₂ and R₃ have the same number of carbon atoms. That means all three alkyl chains have the same length.

It has further been found that very good results concerning the suppression of the aspartimide formation can even be achieved when using comparably short chains with two or three carbon atoms length. Preferably, R₁, R₂ and R₃ are -CH₂-CH₃ or R₁, R₂ and R₃ are -CH₂-CH₂-CH₃.

The present invention is further directed to a method of manufacturing the compounds of formula la and/or Ib. This is preferably done by first providing an aspartic acid with hydrogenizable protecting groups at the N and C terminus. Suitable protecting groups for the N and C terminus are for example benzyloxycarbonyl (Z) and benzyl respectively. A person skilled in the art knows how to introduce such protecting groups. Further information can be found in example 1 and 2.

In a next step the side chain carboxylic acid of the aspartic acid derivative of step a) is reacted with a tertiary alcohol according to formula III

HO-CR₁R₂R₃ Formula III

whereby R₁, R₂, R₃ represent, independently of each other, a linear hydrocarbon chain of two to six carbon atoms length, e.g. -CH₂-CH₃,-CH₂-CH₂-CH₃, -CH₂-CH₂-CH₂-CH₃, -CH₂-CH₂-CH₂-CH₂-CH₃, -CH₂-CH₂-CH₂-C H_{Z}-C H₂-C H₃.

In this step the side chain protecting group is introduced. In a preferred embodiment, the esterification - step is performed with acyl ureas derived from carbodiimides, preferentially dicyclohexylcarbodiimide or diisopropylcarbodiimide in the presence of N,N-dimethylaminopyridine (see Neises, B. and Steglich, W. Angew. Chem.-Int. Ed., 17: 522 - 524 (1978)). After introduction of the side chain protecting group according to the present invention, the hydrogenizable protecting groups at the N and C terminus are split off by hydrogenolysis. This step is known to a person skilled in the art. It is typically performed with hydrogen and a Pd/C catalyst. For use of the aspartic acid derivative in peptide synthesis, finally, a fluorenylmethoxycarbonyl or a benzyloxycarbonyl group is introduced at the N-terminus. Also the introduction of such protecting groups is known to a person skilled in the art of peptide synthesis. It is typically done by N-hydroxysuccinimide activation using Fmoc-OSu or Z-OSu.

The side chain protected aspartic acid derivatives according to the present invention can then be used for solid or liquid phase peptide synthesis. They are typically used in the same manner as e.g. tBu side-chain protected aspartic acid derivatives. The solid phase peptide synthesis is preferably done according to the Fmoc/tBu strategy (Atherton E., et.al., J.Chem. Soc., Chem. Comm., 539 (1978)).

For solid phase synthesis the side chain protected aspartic acid derivatives according to the present invention can for example be used as follows:
In a first step, an amino acid or a peptide bound to a solid phase via its C-terminus is provided. Afterwards, the solid phase bound amino acid or peptide of step a) is reacted with compounds of formula Ia and/or Ib whereby the compounds of formula Ia and/or Ib are attached to the N-terminus. In preferred embodiment, this reaction is performed in the presence of HBTU, TBTU, HATU, TATU, HCTU, TCTU or PyBOP.

Cleavage of the Fmoc group is performed by alkaline treatment. Suitable procedures and reagents are known to a person skilled in the art.

In a preferred embodiment, the alkaline treatment is performed with one or more amines like piperidine, diethylamine, pyrrolidine or DBU, preferably secondary amines, very preferred with 20% piperidine in dimethylformamide.

In a very preferred embodiment, the alkaline treatment of step c) is performed with a mixture comprising one or more amines, preferably secondary amines, e.g. piperidine, and an organic acid like formic acid, acetic acid, or preferably an acidic alcohol like phenol or 2,4-dichlorophenol, (Z)-ethyl cyanoglyoxylate-2-oxime, commercially available as Oxyma Pure (Merck KGaA, Germany, formula IV) or hydroxybenzotriazole.

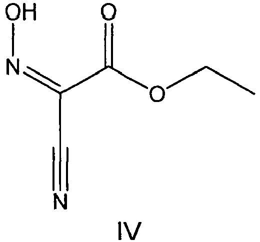

The concentration of the organic acid or the acidic alcohol should be chosen such that it is sub-stoichiometric as compared to the amines.

Preferrably, the molar ratio of the organic acid or the acidic alcohol compared to the amines (acid : amine) is between 0.01 : 1 and 0.4 : 1, preferably between 0.1 : 1 and 0.4 : 1, most preferred between 0.35 : 1 and 0.37 : 1.

It has been found that the addition of the organic acid or preferably the acidic alcohol additionally reduces the formation of aspartimide. It has been further found that ethyl cyanoglyoxylate-2-oxime is especially suitable in molar ratios (acid:amine) between 0.1 : 1 and 0.4 : 1, preferably between 0.35 : 1 and 0.37 : 1. Consequently, the combination of the inventive aspartic acid derivative according to formula la and/or Ib and the addition of a sub-stoichiometric amount of an organic acid or an acidic alcohol when removing the Fmoc protecting group provide optimum conditions for avoiding aspartimide formation.

The invention is also directed to the usage of the inventive aspartic acid side-chain protected derivatives for the peptide synthesis in solution applying the N-terminal Z group. The Z group is cleaved by hydrogenation which doesn't affect the ester bond of the tertiary alcohols. This orthogonality is especially valuable for the preparation of aspartic acid containing peptides in solution (see Subirós-Funosas R. et al., Tetrahedron, 67: 8595-8606, (2011)).

The aspartic acid side-chain protection of the invention is designed to suppress any by-product formed through the base catalyzed aspartimide formation. Any Fmoc SPPS and Z-based solution phase synthesis can be performed or modified with the inventive derivatives. Typically, the said derivatives are stable against the treatment with secondary and tertiary amines like piperidine, pyrrolidine, triethylamine or ethyldiisopropylamine as well as against hydrogenation and are cleaved with strong acids like trifluoroacetic acid. Therefore the derivatives represent a group of semi-permanent side-chain protections. In the present invention the steric hindrance is leveraged by the growing length of straight alkyl chains of tertiary alcohols. It has been found that this homologization builts up a rather flexible bulky structure capable of closely approaching the peptide backbone and thereby hindering aspartimide formation as well as adding enough flexibilty to the system to allow for the efficient coupling of any preceding amino acid.

In a further aspect this invention is directed to the combination of the said aspartic acid derivatives and the usage of slightly acidic alcohols like Oxyma Pure or hydroxybenzotriazole in the Fmoc cleavage solutions. This combination reveals to be a powerful method to reduce the aspartimide formation.

Hence the base catalyzed aspartimide arises especially during Fmoc cleavage the said derivatives are tested in Fmoc SPPS. To investigate the appearance of aspartimide, in the Examples, the hexapeptide sequences of scorpion toxin II (H-Val-Lys-*Asp*-*Gly-*Tyr-Ile-OH) is chosen as model peptide following previous studies and a variant of this hexapeptide (H-Val-Lys-*Asp-Asn*-Tyr-Ile-OH) representing a second Asp-Asn motif with proven susceptibility to aspartimide formation (see Mergler M., et al., J. Peptide Sci., 9: 36-46 (2003)). The impact of multiple Fmoc removals in long peptide stepwise synthesis and harsher Fmoc removal conditions necessary in aggregation prone peptide sequences are systematically examined. Therefore, the hexapeptide resins are on the one hand exposed to prolonged piperidine treatments and on the other hand exposed to 1,8-diazabicyclo[5.4.0]undec-7-en (DBU) treatments. To fully monitor the appearance of aspartimide side reaction all aspartimide derived by-products are identified by LCMS analysis and quantified by HPLC or UPLC analysis. In the Examples the said side-chain protections are compared to the standard compound Fmoc-Asp(OtBu)-OH and Fmoc-Asp(OMpe)-OH, the best commercially available compound, in respect of aspartimide prevention.

As one result no Asp deletion and no deletions of any preceding amino acid could be detected when using the Asp derivatives according to the present invention. It can be assumed that the aspartic acid derivatives provided in this invention do not affect the coupling efficiency in peptide synthesis. Furthermore, the results of the piperidine treatment prove that the Fmoc-Asp(OPhp)-OH derivative according to the invention is significantly more effective in preventing aspartimide formation than the other tested derivatives.

In addition, the Fmoc-Asp(OPhp)-OH derivative nearly inhibits the apartimide side reaction when combined with a Fmoc removal solution of 1 M Oxyma Pure in piperidine/DMF 1:4, even in the Asp-Gly sequence showing the highest susceptibility in previous studies.

The present invention is further described in the following examples which should not be construed as limiting.

### Examples

Materials: All reagents and solvents are purchased in their best available quality either from Merck-Millipore or Sigma-Aldrich. The carbinols 3-ethyl-3-pentanol and 4-(n-propyl)-4-heptanol are purchased from ABCR. The carbinol 5-(n-butyl)-5-nonanol was prepared by standard Grignard reaction.

### Example 1: Synthesis of (2S)-N-((9H-fluoren-9-ylmethyloxy)carbonyl)aspartic acid-4-((3-ethyl)pent-3-yl) ester

### (a) Synthesis of (2S)-N-benzyloxycarbonylaspartic acid 1-benzyl-4-(3-ethyl)pent-3-yl)ester

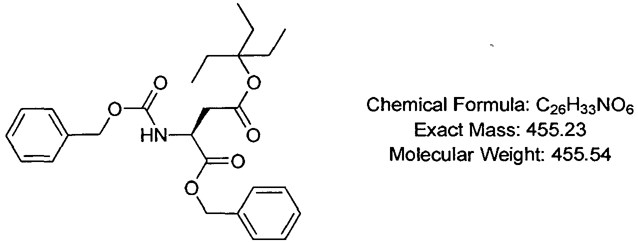

6.7 g (18.7 mmol) of Z-Asp-Obzl (N-benzyloxycarbonylaspartic acid-1-benzylester) are dissolved in 30 ml dichloromethane at room temperature. After addition of 6.6 g (57.1 mmol) of 3-ethyl-pentan-3-ol, the mixture is cooled to 2-3°C and 0.5 g (4.1 mmol) of 4-(N,N-dimethylamino)pyridine is added. Afterwards 4.6 g (22.3 mmol) of dicyclohexyl carbodiimide are added in 4 equal portions over 32 hours. The cooling is stopped after the addition of the first dicyclohexyl carbodiimide portion. The reaction is continued for two days at ambient temperature yielding a thick suspension. The excess of dicyclohexyl carbodiimide is destroyed with acetic acid and the precipitate is filtered off and the solvent evaporated to dryness. The residue is dissolved in a minimum amount of a mixture of toluene/ethylacetate 95:5 and purified by column chromatography using SiO₂ as solid phase and toluene/ethylacetate 95:5 as eluent. After evaporation of the product fractions 8.0 g (17.6 mmol, 94 % of theory) of the title compound were obtained as a colorless solid. ¹H-NMR in CDCl₃ (TMS as internal standard), chemical shift in ppm: 7.41-7.25 (m, 10H, Phenyl), 5.81 (d, 1H, NH), 5.18 (s, 2H, CH2, bzl), 5.10 (s, 2H, CH2, bzl), 4.62(m, CHα, 1H), 3.05 + 2.78 (d, 2H, CH2β), 1.82-1.68 (m, 6H, 3x alkyl-CH2), 0.74-0.68 (t, 9H, 3x alkyl-CH3).

### (b) Synthesis of (2S)-N-((9H-fluoren-9-ylmethyloxy)carbonyl) aspartic acid-4-((3-ethyl)pent-3-yl) ester

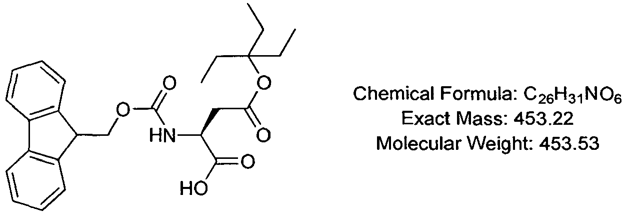

8.0 g (17.6 mmol) of Z-Asp(OEpe)-Obzl (N-benzyloxycarbonylaspartic acid α-benzyl-γ-(3-ethyl)pent-3-yl)ester) are dissolved in 80 ml dioxane at room temperature transferred into an autoclave. 1 g of 5 % Pd/C suspended in 10 ml of water are added and the solution is flushed with nitrogen for inertization. Afterwards the hydrogenation is carried out at a pressure of 0.5 bar hydrogen for 7 hours. The catalyst is filtered off and the resulting filtrate directly applied to the Fmoc protection. For this 38 ml of a 5 % NaHCO₃ solution and a solution of 5.9 g (17.6 mmol) Fmoc-OSu in 50 ml dioxane are added at room temperature. After 4 hours the solvent is removed and the residue purified by extraction. The residual solvent is removed by constant-boiling mixture with methyl-tert-butylether to yield 5.0 g (10.9 mmol, 62 % of theory) of an off white foam. HPLC-purity: 95.2 area %, retention time 22.7 min (column: ACE C18-AR, 5 µm, 150x4.6 mm, linear acetonitrile gradient of 0 % to 100 % B over 30 min, buffer A = 2 % acetonitrile and 0.1 % trifluoroacetic acid in water, buffer B = 0.1 % trifluoroacetic acid in acetonitrile, flow 1 ml/min); ¹H-NMR in CDCl₃ (TMS as internal standard), chemical shift in ppm: 7.78 (d, 2H, Fmoc), 7.62 (m, 2H, Fmoc), 7.45-7.25 (m, 4H, Fmoc), 5.82 (d, 1 H, NH), 4.68 (d, 1 H, CH, Fmoc), 4.48-4.28 (m, 2H, Fmoc), 4.24-4.17 (m, 1H, CHα), 3.05 + 2.78 (d, 2H, CH2β), 1.91-1.78 (m, 6H, 3x alkyl-CH2), 0.94-0.75 (t, 9H, 3x alkyl-CH₃); ESI-MS: monoisotopic M_{W calc}. = 453.2, M_{W} [M+Na]⁺ = 476.1.

### Example 2: Synthesis of (2S)-N-((9H-fluoren-9-ylmethyloxy)carbonyl)aspartic acid-4-(4-(n-propyl)hept-4-yl) ester

### (a) Synthesis of (2S)-N-benzyloxycarbonylaspartic acid 1-benzyl-4-(4-(n-propyl)hept-4-yl) ester

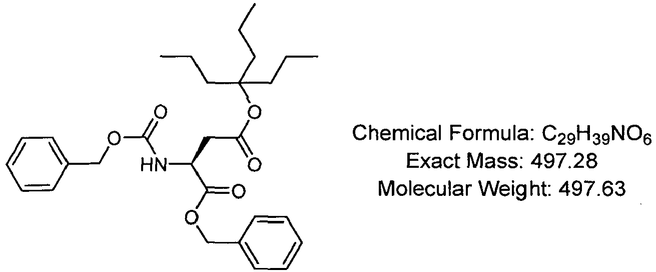

25.0 g (70.0 mmol) of Z-Asp-Obzl (N-benzyloxycarbonylaspartic acid-1-benzylester) are dissolved in 100 ml dichloromethane at room temperature. After addition of 12.2 g (77.0 mmol) of 4-(n-propyl)-4-heptanol, the mixture is cooled to 2-3°C and 1.7 g (14.0 mmol) of 4-(N,N-dimethylamino)pyridine is added. Afterwards 10.6 g (84.0 mmol) of diisopropyl carbodiimide are added in 4 equal portions over 48 hours. The cooling is removed after the addition of the first dicyclohexyl carbodiimide portion. The reaction is continued for two days at ambient temperature. The excess of diisopropyl carbodiimide is destroyed with acetic acid and the reaction mixture is filtered over silica gel and the product fractions evaporated to dryness. The residue is purified by column chromatography using SiO₂ as solid phase and toluene/ethylacetate 95:5 as eluent. After evaporation of the product fractions 19.4 g (39.0 mmol, 56 % of theory) of the title compound were obtained as a colorless foam. ¹H-NMR in CDCl₃ (TMS as internal standard), chemical shift in ppm: 7.41-7.25 (m, 10H, Phenyl), 5.79 (d, 1H, NH), 5.18 (m, 2H, CH2, bzl), 5.10 (s, 2H, CH2, bzl), 4.62(m, CHα, 1H), 3.00 + 2.71 (d, 2H, CH2β), 1.77-1.61 (t, 6H, 3x alkyl-CH2), 1.32-1.08 (m, 6H, 3x alkyl-CH2), 0.95-0.78 (t, 9H, 3x alkyl-CH3).

### (b) Synthesis of (2S)-N-((9H-fluoren-9-ylmethyloxy)carbonyl) aspartic acid-4-(4-(n-propyl)hept-4-yl) ester

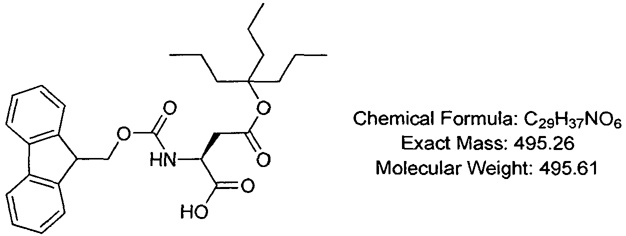

18.0 g (36.2 mmol) of Z-Asp(OPhp)-Obzl (N-benzyloxycarbonylaspartic acid α-benzyl-y-(4-(n-propyl)hept-4-yl)ester) are dissolved in 200 ml dioxane at room temperature transferred into an autoclave. 2.8 g of 5 % Pd/C suspended in 20 ml of water are added and the solution is flushed with nitrogen for inertization. Afterwards the hydrogenation is carried out at a pressure of 0.5 bar hydrogen for 7 hours. The catalyst is filtered off and the resulting filtrate directly applied to the Fmoc protection. Therefore 20 ml of a 10 % NaHCO₃ solution and 13.5 g (40.0 mmol) Fmoc-OSu are added at room temperature. After 6 hours the solvent is removed and the residue purified by extraction. The residual solvent is removed by constant-boiling mixture with methyl-tert-butylether. The resulting oil is purified bv RP-HPLC. The product fractions are freeze dried to yield 1.4 g (2.8 mmol, 8 % of theory) of a white foam. HPLC-purity: 97.1 area %, retention time 25.4 min (column: ACE C18-AR, 5 µm, 150x4.6 mm, linear acetonitrile gradient of 0 % to 100 % B over 30 min, buffer A = 2 % acetonitrile and 0.1 % TFA in water, buffer B = 0.1 % TFA in acetonitrile, flow 1 ml/min); ¹H-NMR in CDCl₃ (TMS as internal standard), chemical shift in ppm: 7.82 (d, 2H, Fmoc), 7.63 (m, 2H, Fmoc), 7.25-7.45 (m, 4H, Fmoc), 5.87 (d, 1H, NH), 4.65 (m, 1H, CH, Fmoc), 4.28-4.48 (m, 2H, Fmoc), 4.17-4.25 (m, 1H, CHα), 2.72 + 3.05 (2xd, 2H, CH2β), 1.68-1.85 (m, 6H, 3x alkyl-CH2), 1.18-1.37 (m, 6H, 3x alkyl-CH2), 0.82-1.01 (t, 9H, 3x alkyl-CH₃); ESI-MS: monoisotopic M_{W calc}. = 495.26, M_{W} [M+Na]⁺ = 518.26.

### Example 3: Synthesis of (2S)-N-((9H-fluoren-9-ymethyloxy)carbonyl)aspartic acid-4-(5-(n-butyl)nona-5-yl ester

### (a) Synthesis of (2S)-N-benzyloxycarbonylaspartic acid 1-benzyl-4-(5-(n-butyl)nonan-5-yl) ester

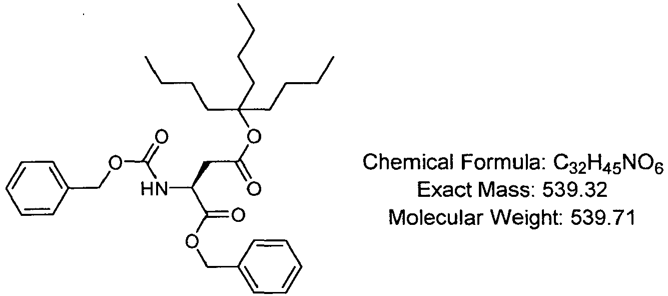

14.3 g (40.0 mmol) of Z-Asp-OBzl (N-benzyloxycarbonylaspartic acid-1-benzylester) are dissolved in 60 ml dichloromethane at room temperature. After addition of 23.2 g (116 mmol) of 5-n-butyl-5-nonanol, the mixture is cooled to 0°C and the first portion of 4.1 g (20.0 mmol) of dicyclohexyl carbodiimide is added. After 15 minutes preactivation at 0°C 1.0 g (8.0 mmol) of 4-(N,N-dimethylamino)pyridine is added. The reaction is continued at ambient temperature over four days by adding daily 1.65 g (8.0 mmol) of dicyclohexyl carbodiimide over the first three days yielding a thick suspension. The excess of dicyclohexyl carbodiimide is destroyed with acetic acid. The suspension is diluted with 500 ml toluene/ethyl acetate (95:5) and filtered over 40 g of silica gel. After evaporation to dryness the residue is dissolved in a minimum amount of a mixture of cyclohexane/ethylacetate 9:1 and purified by column chromatography using 500 g SiO₂ as solid phase and cyclohexane/ethylacetate 9:1 as eluent. After evaporation of the product fractions 15.3 g (28.4 mmol, 71 % of theory) of the title compound are obtained as an off white liquid wax. ¹H-NMR in CDCl₃ (TMS as internal standard), chemical shift in ppm: 7.39-7.25 (m, 10H, Phenyl), 5.82 (d, 1H, NH), 5.07-5.25 (m, 2H, CH2, bzl), 5.10 (s, 2H, CH2, bzl), 4.54-4.65 (m, 1 H, CHα), 2.78 + 3.01 (2xd, 2H, CH2β), 1.65-1.81 (m, 6H, 3x alkyl-CH2), 1.09-1.34 (m, 12H, 6x alkyl-CH2), 0.83-0.96 (t, 9H, 3x alkyl-CH₃)).

### (b) Synthesis of (2S)-N-((9H-fluoren-9-ylmethyloxy)carbonyl) aspartic acid-4-(5-(n-butyl)nonan-5-yl)) ester

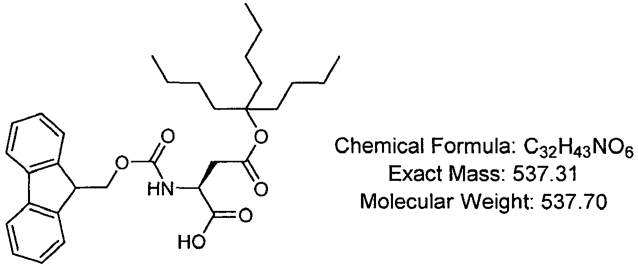

14.6 g (26.9 mmol) of Z-Asp(OBno)-Obzl (N-benzyloxycarbonylaspartic acid α-benzyl-y-(5-(n-butyl)nonan-5-yl)ester) are dissolved in 120 ml dioxane at room temperature transferred into an autoclave. 2.4 g of 10 % Pd/C are added and the solution is flushed with argon for inertization. Afterwards the hydrogenation is carried out at a pressure of 0.5 bar hydrogen for 27 hours. The pH is kept between 7.5 to 8 during the hydrogenation by the addition of sodiumcarbonate. The catalyst is filtered off and the resulting filtrate is directly applied to Fmoc protection. To this end the filtrate is diluted with 300 ml dioxane/water (8:2) and the pH adjusted to 8 with 2 g of sodiumbicarbonate. 10.0 g (29.6 mmol) Fmoc-OSu are added at room temperature and the mixture left to react overnight. The reaction mixture is purified by extraction and the combined organic layers evaporated. After evaporation to dryness the residue is dissolved in a minimum amount of a mixture of dichloromethane/methanol 98:2 and purified by column chromatography using 500 g SiO₂ as solid phase and dichloromethane/ methanol 98:2 as eluent. After evaporation of the product fractions 10.0 g of the title compound were obtained. To further improve purity the material is purified by a second column chromatography run using 300 g SiO₂ as solid phase and cyclohexane/ethyl acetate/acetic acid (6:4:0.005) as eluent. After evaporation of the product fractions 6.5 g of the title compound are obtained as an off-white wax (12.1 mmol, 45 % of theory). (HPLC-purity: 99.0 area %, retention time 27.6 min (column: ACE C18-AR, 5 µm, 150x4.6 mm, linear acetonitrile gradient of 0 % to 100 % B over 30 min, buffer A = 2 % acetonitrile and 0.1 % trifluoroacetic acid in water, buffer B = 0.1 % trifluoroacetic acid in acetonitrile, flow 1 ml/min); ¹H-NMR in CDCl₃ (TMS as internal standard), chemical shift in ppm: 10.33 (s, 1H, COOH), 7.75 (d, 2H, Fmoc), 7.55 (m, 2H, Fmoc), 7.19-7.43 (m, 4H, Fmoc), 5.90 (d, ¹H, NH), 4.66 (d, 1 H, CH, Fmoc), 4.26-4.52 (m, 2H, Fmoc), 4.14-4.26 (m, 1H, CHα), 3.05 + 2.81 (d, 2H, CH2β), 1.62-1.83 (m, 6H, 3x alkyl-CH2), 1.07-1.38 (m, 12H, 6x alkyl-CH2), 0.79-0.95 (t, 9H, 3x alkyl-CH₃); ESI-MS: monoisotopic M_{W calc}. = 537.3, M_{W} [M+Na]⁺ = 560.3.

### Example 4: Synthesis of H-Val-Lys-Asp-Gly-Tyr-Ile-OH (VKDGYI) using Fmoc-Asp(OEpe)-OH, Fmoc-Asp(OPhp)-OH, Fmoc-Asp(OMpe)-OH and Fmoc-Asp(OtBu)-OH

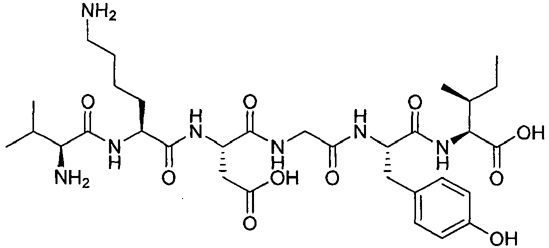

The solid phase peptide synthesis is carried out following the Fmoc/tBu strategy (Atherton E., et.al., J.Chem. Soc., Chem. Comm., 539 (1978)). 0.44 g of Fmoc-Ile-Wang resin (0.54 mmol/g) are used in an ABI 431 for automated peptide synthesis employing HBTU/ ethyldiisopropylamine (3.6 eq./8.0 eq.) activation for amide bond formation and a solution of 20 % piperidine in dimethylformamide for Fmoc removal. The corresponding amino acids Fmoc-Val-OH, Fmoc-Lvs(Boc)-OH, Fmoc-Asp(OtBu/OMpe/ OEpe/OPhp)-OH, Fmoc-Gly-OH and Fmoc-Tyr(tBu)-OH are used in a four fold excess. 50 mg samples of the resulting peptide resins are exposed to 0.5 ml 95% TFA in water for 1.5 hours, the resin filtered off and the peptide filtrate precipitated in diisopropylether to yield the crude fully deprotected peptides.

Further 50 mg peptide resin samples are treated with a solution of 20 % piperidine in dimethylformamide over 18 hours to simulate multiple Fmoc removals. Afterwards the peptide resin samples are exposed to 95% TFA in water for final cleavage in analogy to the above described protocol and all relevant impurities are identified by MS analysis (Xevo Waters Q-TOF-1, lock mass = 556.277 (leu-enkephaline)) and quantified by HPLC analysis (HPLC conditions: Halo Peptide 2,7 µm (150x4.6 mm) at 40 °C; water acetonitrile gradient of 5 % to 35 % B over 30 min where buffer A is 0.1% TFA and 2 % acetonitrile in water and buffer B is 0.1 % TFA in acetonitrile, flow 1 ml/min).

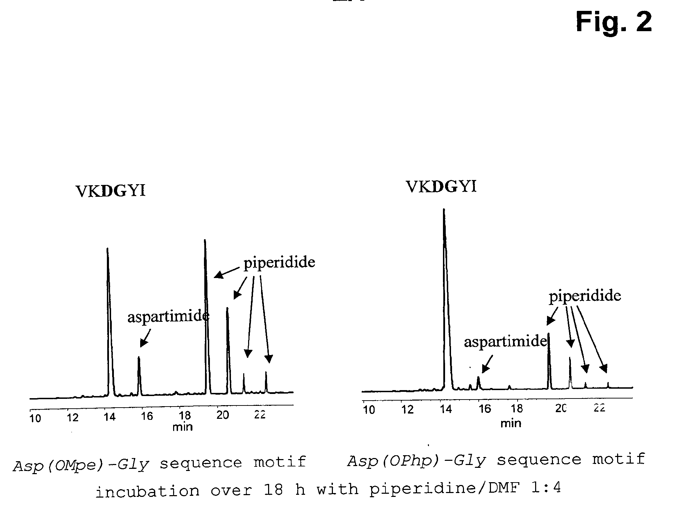

As expected the prolonged exposure of the peptide resins to piperidine exhibited different levels of decompositions in respect to the applied side-chain protection. Figure 2 illustrates the corresponding HPLC traces for the best commercially available Fmoc-Asp(OMpe)-OH derivative in comparison to Fmoc-Asp(OPhp)-OH. In table 1 the amounts of by-products after prolonged piperidine treatment are summarized. The treatment with piperidine over 18 hours equals 108 Fmoc removal cycles à 10 minutes.

Therefore this experiment correlates to a Fmoc SPPS assembly of an 100mer after introducing the aspartic acid residue.

**Table 1: Results after 110 Fmoc removal cycles using piperidine, target peptide = VKDGYI, the aspartimide (Asu) derived by-products are the aspartimide itself and the sum of piperidides**

| **Asp(OR) R =** | **target [%]** | **Asu [%]** | **piperidides [%]** | **Asu per 10' cycle [%]** | **Half-life in 10' cycles** |
|---|---|---|---|---|---|
| *tBu* | 8.0 | 6.4 | 85.6 | 2.3 | 30 |
| *Mpe* | 42.3 | 6.7 | 51.0 | 0.8 | 90 |
| *Epe* | 64.4 | 4.1 | 31.5 | 0.4 | 175 |
| *Php* | 80.6 | 2.9 | 16.5 | 0.2 | 356 |

As seen in table 1 the half-life of the target peptide containing the most susceptible Asp-Gly sequence is increased by more than 10 fold using Php protection instead of tBu and by 4 fold in respect of Mpe. Of special interest is the comparison of the Epe vs. the Mpe protection since their main difference is a symmetric vs. an asymmetric shield of the γ-carboxy group of the aspartyl residue. Epe protection minimizes the aspartimide formation by a factor two compared to Mpe which is a first evidence for the advantageous properties of the side chain protections presented in this invention to overcome aspartimide formation.

### Example 5: Synthesis of H-Val-Lys-Asp-Asn-Tyr-Ile-OH (VKDNYI) using Fmoc-Asp(OEpe)-OH, Fmoc-Asp(OPhp)-OH, Fmoc-Asp(OBno)-OH, Fmoc-Asp(OMpe)-OH and Fmoc-Asp(OtBu)-OH

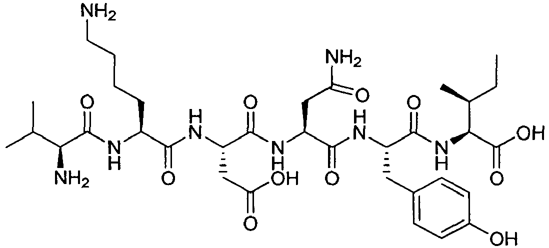

Automated peptide synthesis, piperidine treatments of the resin samples and final TFA cleavages are performed as described in the protocols of example 4. In addition, 50 mg peptide resin samples are treated with a solution of DBU/piperidine/DMF (1:19:79) over 225 minutes to investigate the aspartimide formation at harsher Fmoc deprotection conditions often used for complete Fmoc removals in aggregation prone sequences.

The resulting peptide samples are analysied and all relevant impurities identified by UPLC-MS analysis (Xevo Waters Q-TOF-1, lock mass = 556.277 (leu-enkephaline), LC-conditions: CHS 18 1,7 µm (100x2.1 mm) at 40°C; water acetonitrile gradient of 5% to 35 % B over 10 min where buffer A is 0.1 % formic acid in water and buffer B is 0.1 % formic acid in acetonitrile, flow 0.6 ml/min).

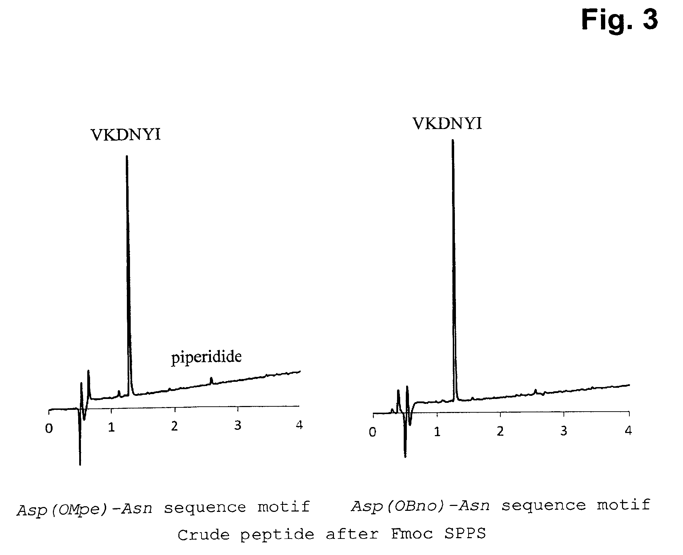

Figure 3 shows the UPLC traces of the crude peptides directly after the synthesis for the most sterical demanding Asp(OBno) derivative in comparison to Asp(OMpe). To the limit of detection no Asp deletion and no deletions of a proceding amino acid are detected.

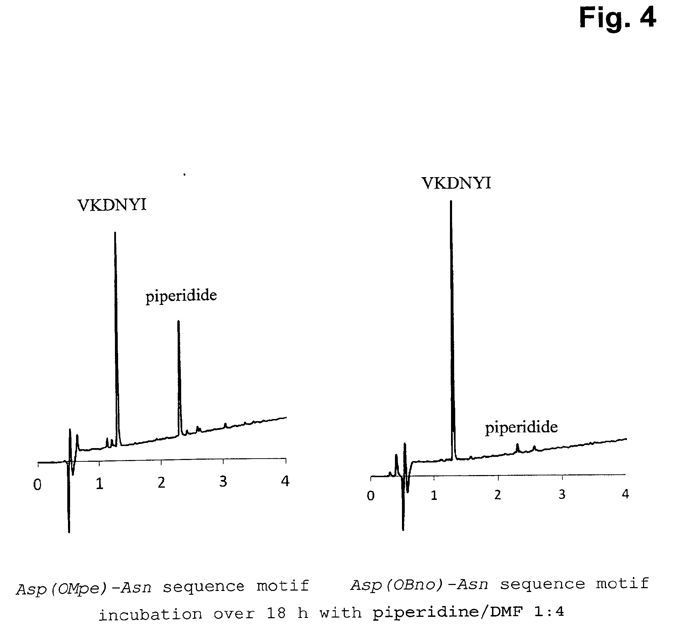

In table 2 the amounts of by-products formed after the prolonged exposure of the peptide resins to piperidine (110 Fmoc removal cycles) are summarized for all side-chain protections used in this invention. Figure 4 shows the corresponding UPLC traces for the Fmoc-Asp(OMpe)-OH derivative in comparison to Fmoc-Asp(OBno)-OH.

**Table 2: Results after 110 Fmoc removal cycles using piperidine, target peptide = VKDNYI, the aspartimide (Asu) derived by products are the aspartimide itself and the sum of piperidides**

| **Asp(OR) R =** | **target [%]** | **D/L-Asu [%]** | **piperidides [%]** | **Asu per 10' cycle** | **Half-life in cycles** |
|---|---|---|---|---|---|
| *tBu* | 16.1 | 4.9 | 79.0 | 1.6 | 40 |
| *Mpe* | 58.2 | 4.3 | 37.5 | 0.5 | 140 |
| *Epe* | 81.0 | 3.0 | 16.0 | 0.2 | 365 |
| *Php* | 90.8 | 1.0 | 8.2 | 0.09 | 800 |
| *Bno* | 95.5 | 0.9 | 3.6 | 0.04 | 1670 |

In analogy to example 4, the Asp-Gly sequence motif, the results for the Asp-Asn motif exhibited different levels of decompositions in respect to the applied side-chain protection. The Bno protecting group displaying the most sterically demanding group in this run does not impose any hindrance to the peptide synthesis used under standard conditions, but is capable to limit the aspartimide side reaction during the Fmoc removals to a rate of 0.05% per cycle with a half-life of more than 1500 cycles. In comparison, the best commercially available Fmoc-Asp(OMpe)-OH derivative yielded ten times more aspartimide per Fmoc removal cycle with a half-life of 140 cycles.

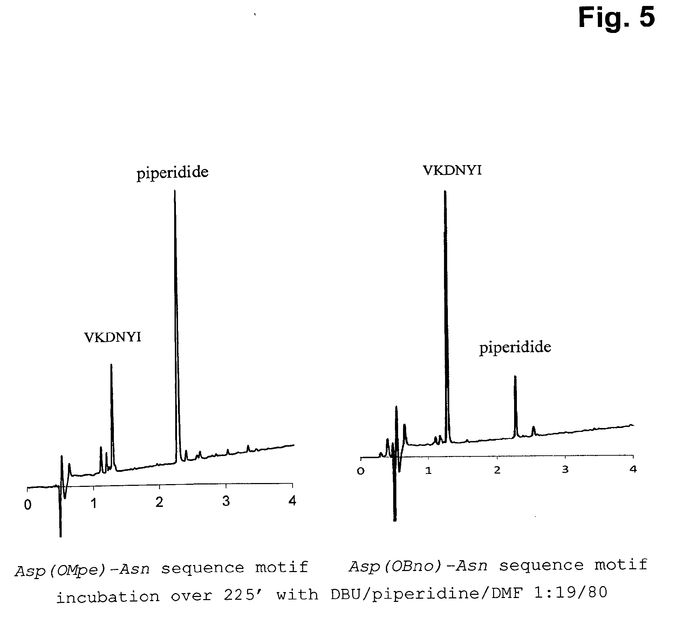

In table 3 the amounts of by-products formed after prolonged DBU treatments are summarized for all side-chain protections used in this invention. Figure 5 shows the corresponding UPLC traces for the Fmoc-Asp(OMpe)-OH derivative in comparison to Fmoc-Asp(OBno)-OH. Since the DBU mediated Fmoc removals are much quicker than the piperidine mediated, this experiment again represents a Fmoc SPPS assembly of an 100mer after introducing the aspartic acid residue using exclusively DBU for Fmoc cleavage.

**Table 3: Results after 110 Fmoc removal cycles using DBU, target peptide = VKDNYI, the aspartimide (Asu) derived by-products are the aspartimide itself and the sum of piperidides**

| **Asp(OR) R =** | **target [%]** | **D/L-Asu [%]** | **piperides [%]** | **Asu per 2' cycle** | **Half-life in cycles** |
|---|---|---|---|---|---|
| *tBu* | 0.3 | 2.6 | 97.1 | 5.2 | 13 |
| *Mpe* | 18.8 | 7.5 | 73.7 | 1.5 | 46 |
| *Epe* | 57.1 | 5.6 | 37.3 | 0.5 | 138 |
| *Php* | 71.0 | 3.3 | 25.7 | 0.4 | 192 |
| *Bno* | 74.5 | 3.5 | 22.0 | 0.3 | 263 |

In case of aggregation prone sequences incomplete coupling efficiency is often accompanied by incomplete Fmoc removals during standard SPPS.

Harsher Fmoc removal conditions need to be employed to solve the later issue. However, DBU treatments give rise to aspartimide derived impurities in all Asp-Xaa sequence motifs (see Mergler M., et al., J. Peptide Sci., 9: 36-46 (2003)). This fact strongly limits the use of harsher Fmoc removal conditions in Fmoc SPPS. In our experiments the Bno protection represents the best solution for this situation reducing the aspartimide formation to less than 0.3% per 2 minute Fmoc removal cycle with a half-life of 260 cycles. Thereby Bno for the first time allows to weigh up the advantages of complete Fmoc removals versus the associated risk of DBU mediated aspartimide formation.

### Example 6: Synthesis of H-Val-Lys-Asp-Arg-Tyr-Ile-OH (VKDRYI) using Fmoc-Asp(OEpe)-OH, Fmoc-Asp(OPhp)-OH, Fmoc-Asp(OBno)-OH Fmoc-Asp(OMpe)-OH and Fmoc-Asp(OtBu)-OH

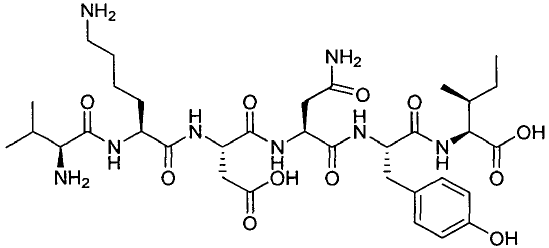

Automated peptide synthesis, piperidine treatments of the resin samples and final TFA cleavages are performed as described in the protocols of example 4. In addition, to evaluate the advantage of the protecting groups used in this invention in heat accelerated SPPS we treat 50 mg peptide resin samples with a solution of 20% piperidine in DMF at 60°C over 200 minutes. Microwave assisted peptide synthesis conditions are often used to shorten peptide synthesis times and increase coupling reaction and Fmoc deprotection.

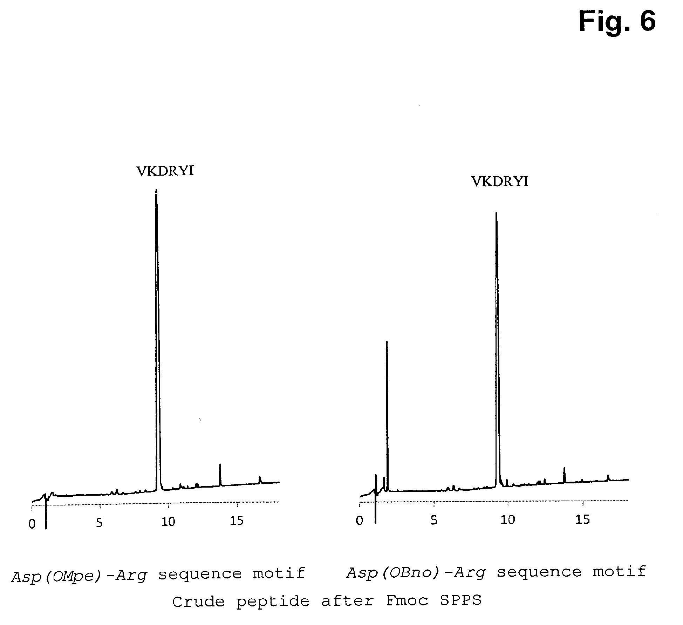

All relevant impurities are quantified by HPLC analysis analysis (HPLC conditions: Kinetex XB-C18 2,6 µm (150x4.6 mm) column at 50 °C; water acetonitrile gradient of 5 % to 30 % B over 18 min where buffer A is 20 mM ammonium formiate and 2 % acetonitrile in water and buffer B is 20 mM ammonium formiate in acetonitrile, flow 1 ml/min) and identified by MS analysis (Xevo Waters Q-TOF-1, lock mass = 556.277 (leu-enkephaline)) Figure 6 shows the HPLC traces of the crude peptides directly after the synthesis for the most sterical demanding Asp(OBno) derivative in comparison to Asp(OMpe). To the limit of detection no Asp deletion and no deletions of a proceeding amino acid are detected.

In table 4 the amounts of by-products formed after the prolonged exposure of the peptide resins to piperidine (110 Fmoc removal cycles) are summarized for all side-chain protections used in this invention.

**Table 4: Results after 110 Fmoc removal cycles using piperidine, target peptide sequence = VKDRYI, the aspartimide (Asu) derived by-products are the aspartimide itself and the sum of piperidides**

| **Asp(OR) R =** | **target [%]** | **D/L-Asu [%]** | **piperidides [%]** | **Asu per 10' cycle** | **Half-life in cycles** |
|---|---|---|---|---|---|
| *tBu* | 24.7 | 16.9 | 58.4 | 1.3 | 55 |
| *Mpe* | 64.2 | 6.9 | 28.9 | 0.4 | 173 |
| *Epe* | 86.7 | 3.90 | 9.4 | 0.1 | 539 |
| *Php* | 91.3 | 3.2 | 5.5 | 0.08 | 845 |
| *Bno* | 93.8 | 2.6 | 3.6 | 0.06 | 1202 |

The Asp-Arg sequence motif showed in complete accordance to the previous results reduced levels of decompositions especially when a symmetric shield is applied to the side-chain protection. Although the Bno protecting group displays the most sterically demanding group it does not impose any hindrance to the peptide synthesis used under standard conditions. The optimized steric shield on the other hand is very well capable to suppress the aspartimide side reaction during the Fmoc removals to less than 0.06% per cycle with a half-life per cycle of greater 1000. In comparison, the best commercially available Fmoc-Asp(OMpe)-OH derivative yielded approximately seven times more aspartimide per Fmoc removal cycle with a half-life of approx. 170 cycles.

The application of microwave heating to SPPS is accelerating coupling and deprotection reactions leading to shorter cycle times, higher repetitive yields, and ultimately purer peptides. On the other hand, microwave energy can also lead to increased levels of aspartimide by-products. To overcome that drawback we evaluated the advantage of the side-chain protections used in this invention by exposing the peptide resins to Fmoc removal cycles at 60 degrees Celsius.

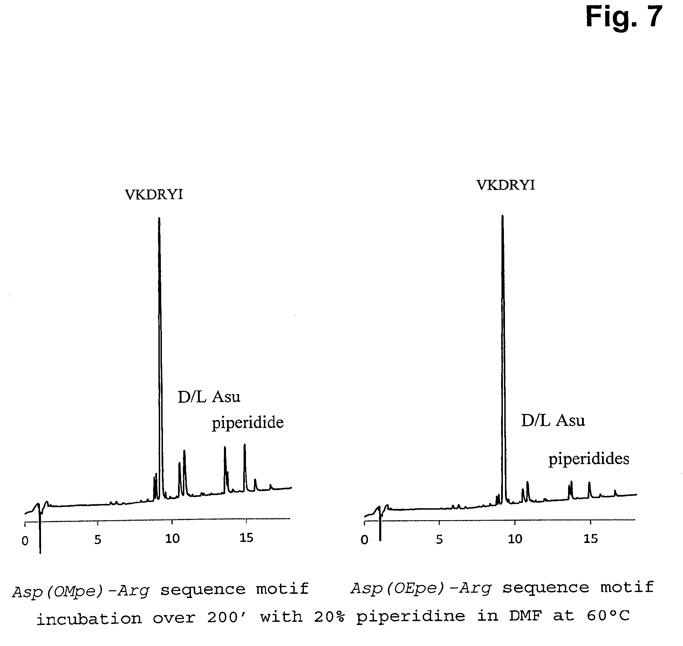

In table 5 the amounts of by-products formed after the prolonged exposure of the peptide resins to piperidine at 60 degree Celsius (100 Fmoc removal cycles) are summarized for all side-chain protections used in this invention. Figure 7 shows the corresponding HPLC traces for the Fmoc-Asp(OMpe)-OH derivative in comparison to Fmoc-Asp(OBno)-OH.

**Table 5: Results after 100 Fmoc removal cycles using piperidine at 60°C, target peptide sequence = VKDRYI, the aspartimide (Asu) derived by-products are the aspartimide itself and the sum of piperidides**

| **Asp(OR) R =** | **target [%]** | **D/L-Asu [%]** | **piperidides [%]** | **Asu per 10' cycle** | **Half-life in cycles** |
|---|---|---|---|---|---|
| *tBu* | 30.3 | 28.7 | 41.0 | 1.2 | 58 |
| *Mpe* | 63.5 | 16.9 | 19.6 | 0.5 | 153 |
| *Epe* | 85.4 | 7.8 | 6.8 | 0.2 | 439 |
| *Php* | 87.8 | 7.1 | 5.1 | 0.1 | 532 |
| *Bno* | 90.8 | 4.9 | 4.3 | 0.1 | 718 |

First of all the results show that the Asp-Arg sequence is highly susceptible to aspartimide formation under microwave heating. Using the asymmetric Mpe protecting group 0.5% aspartimide by-products per cycle are formed which is represented by a half-life of 153 cycles whereas the symmetrical Epe group leads only to 0.2% of aspartimide formation with a half-life of 439 cycles. This may be attributed to the weak point of the Mpe protection being composed Epe group leads only to 0.2% of aspartimide formation with a half-life of 439 of only two ethyl and one methyl branch. However, due to the Brownian motion differences within the homologs are becoming marginal at 60 degree Celsius

This example demonstrates that the optimized steric shields in the side-chain protections used in this invention can be applied to efficiently suppress aspartimide side reactions, even at elevated temperatures, for the sensitive Asp-Arg sequence motif.

### Example 7: Synthesis of H-Val-Lys-Asp-Gly-Tyr-Ile-OH (VKDGYI) using Fmoc-Asp(OEpe)-OH, Fmoc-Asp(OPhp)-OH, Fmoc-Asp(OMpe)-OH and Fmoc-Asp(OtBu)-OH as well as 1 M Oxyma Pure in the Fmoc cleavage solution

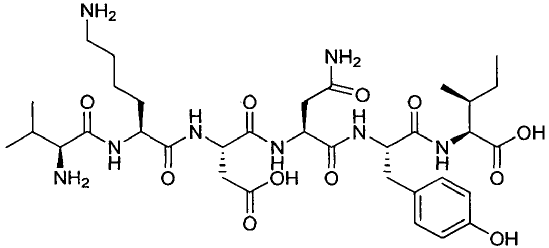

50 mg samples of the corresponding aspartic acid derivatives of example 4 are treated over 18 hours with a solution of 1 M Oxyma Pure and 20 % piperidine in dimethylformamide. The peptides are cleaved off the resin with 95 % TFA and analysed with HPLC as well as MS to identify all aspartimide related products (conditions see example 4). The results are summarized in table 6:

**Table 6: Results after 110 Fmoc removal cycles using an acidic modifier in the piperidine mediated Fmoc removal cocktails, target peptide = VKDG YI, the aspartimide derived by-products are the aspartimide itself and the sum of piperidides**

| **Asp(OR)** | **Oxyma** | **target** | **Asu** | **piperidides** | |
|---|---|---|---|---|---|
| | **Asu per** | | | | |
| **R=** | **Pure** | **[%]** | **[%]** | **[%]** | **10' cycle** |
| tBu | + | 61.1 | 8.8 | 30.1 | 0.45 |
| | - | 8.0 | 6.4 | 85.6 | 2.3 |
| Mpe | + | 84.2 | 3.8 | 12.0 | 0.16 |
| | - | 42.3 | 6.7 | 51.0 | 0.8 |
| Epe | + | 85.7 | 2.6 | 11.7 | 0.14 |
| | - | 64.4 | 4.1 | 31.5 | 0.4 |
| **Php** | **+** | **95.4** | **1.3** | **3.3** | **0.04** |
| | - | 80.6 | 2.9 | 16.5 | 0.2 |

Besides to the fact that the addition of Oxyma Pure to the standard piperidine Fmoc removal cocktail generally decreases the occurrence of aspartimide side reaction (see R. Subiros-Funosas, et al., Chem. Eur. J., 2009, 9394) to our surprise a synergistic effect is observed combining this method with side-chain protections of the present invention especially in case of the Php protection. Since the peptide resin treatments again resemble 110 Fmoc deprotection cycles the combination of Asp(OPhp) with 1 M Oxyma Pure in 20 % piperidine leads to a negligible level of 0.05 % of aspartimide derived by-products per Fmoc removal with a half-life greater than 1500 cycles. Since this applies for the most susceptible Asp-Gly sequence motif the Asp(OPhp) protection in combination with a mild Fmoc deprotection method represents an highly efficient solution for the aspartimide problem in Fmoc SPPS in general. However, it is important to note that Fmoc removal cocktails containing an acidic modifier are sometimes too weak to drive the Fmoc cleavage to completion within 10 minutes. Prolonged exposure is often necessary.

### Abbreviations

- Fmoc-OSu: N-(9-Fluorenylmethoxycarbonyloxy)-succinimid
- Asp(OPhp): aspartic acid-4-(4-(n-propyl)hept-4-yl-) ester
- Asp(OMpe): aspartic acid-4-(3-methylpent-3yl-) ester
- Asp(OEpe): aspartic acid-4-(3-ethylpent-3-yl-) ester
- Php: 4-(n-propyl)hept-4-yl-
- Mpe: 3-methylpent-3yl-
- Epe: 3-ethylpent-3-yl-
- tBu: *tert*-butyl
- DBU: 1,8-diazabicyclo[5.4.0]undec-7-en
- HBTU: 2-(1H-Benzotriazole-1-yl)-1,1,3,3-tetramethyluronium hexafluorophosphate
- HATU: 2-(7-Aza-1-H-benzotriazole-1-yl)-1,1,3,3-tetramethylaminium hexafluorophosphate
- HCTU: 2-(6-Chloro-1-H-benzotriazole-1-yl)-1,1,3,3-tetramethylaminium hexafluorophosphate
- PyBOP: Benzotriazole-1-yl-oxy-tris-pyrrolidino-phosphonium hexafluorophosphate
- TBTU: 2-(1 H-Benzotriazole-1-yl)-1,1,3,3-tetramethyluronium tetrafluoroborate
- TATU: 2-(7-Aza-1-H-benzotriazole-1-yl)-1,1,3,3-tetramethylaminium tetrafluoroborate
- TCTU: 2-(6-Chloro-1-H-benzotriazole-1-yl)-1,1,3,3-tetramethylaminium tetrafluoroborate

## Claims

1. Compounds of formula la and/or Ib 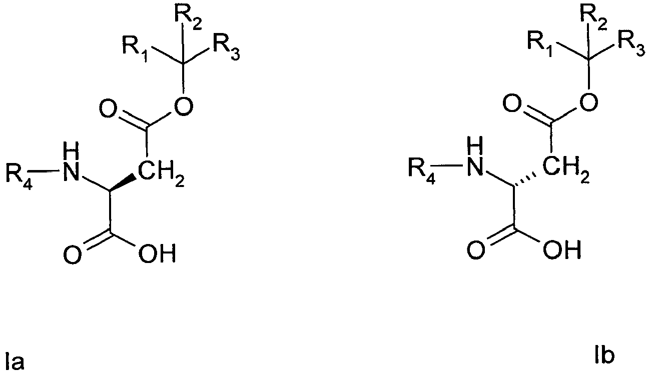 wherein
R₁, R₂, R₃ represent, independently of each other, a linear hydrocarbon chain of two to six carbon atoms length,
R₄ represents benzyloxycarbonyl, 9-fluorenylmethoxycarbonyl or H.

2. Compound according to claim 1, **characterized in that** R₁, R₂, R₃ all have the same number of carbon atoms.

3. Compound according to claim 1 or 2, **characterized in that** R₁, R₂, R₃ are -CH₂-CH₃ or -CH₂-CH₂-CH₃.

4. A method of manufacturing the compound of formula la and/or Ib by
a) Providing an aspartic acid with hydrogenizable protecting groups at the N and C terminus
b) Esterification of the side chain carboxylic acid with the tertiary alcohol according to formula III
HO-CR₁R₂R₃ formula III
whereby R₁, R₂, R₃ represent, independently of each other, a linear hydrocarbon chain of two to six carbon atoms length
c) Hydrogenolysis of the hydrogenizable protecting groups at the N and C terminus
d) Introduction of a fluorenylmethoxycarbonyl or a benzyloxycarbonyl group at the N-terminus.

5. Method according to claim 4, **characterized in that** the esterification of step b) is performed with a carbodiimide.

6. A method for peptide synthesis by
a) Providing an amino acid or a peptide bound to a solid phase via its C-terminus
b) Reacting the solid phase bound amino acid or peptide of step a) with a compound of formula la and/or Ib as defined in claim 1, wherein R4 is 9-fluorenylmethoxycarbonyl (Fmoc) whereby the compound of formula la and/or Ib is attached to the N-terminus
c) Cleavage of the Fmoc group by alkaline treatment.

7. Method according to claim 6 **characterized in that** the alkaline treatment of step c) is performed with one or more secondary amines.

8. Method according to claim 6 or 7 **characterized in that** the alkaline treatment of step c) is performed with a mixture comprising one or more secondary amines and an organic acid and/or an acidic alcohol.

9. Method according to one or more of claims 6 to 8 **characterized in that** the reaction in step b) is performed in the presence of HBTU, TBTU, HATU, TATU, HCTU, TCTU or PyBOP.

## Patentansprüche

1. Verbindungen der Formel la und/oder Ib 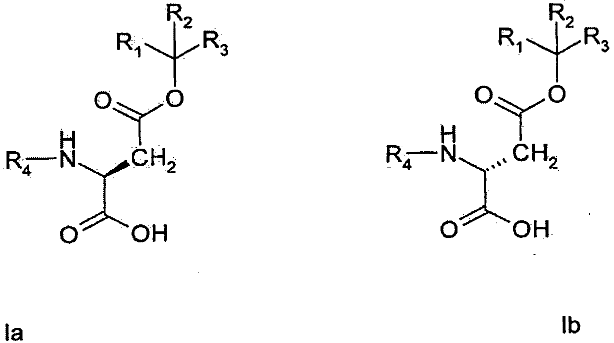 in denen
R₁, R₂, R₃ unabhängig voneinander eine lineare Kohlenwasserstoffkette mit einer Länge von zwei bis sechs Kohlenstoffatomen darstellen,
R₄ Benzyloxycarbonyl, 9-Fluorenylmethoxycarbonyl oder H darstellt.

2. Verbindung nach Anspruch 1, **dadurch gekennzeichnet, dass** R₁, R₂, R₃ alle die gleiche Anzahl Kohlenstoffatome aufweisen.

3. Verbindung nach Anspruch 1 oder 2, **dadurch gekennzeichnet, dass** R₁, R₂, R₃ -CH₂-CH₃ oder -CH₂-CH₂-CH₃ bedeuten.

4. Verfahren zur Herstellung der Verbindung der Formel la und/oder Ib durch
a) Bereitstellen einer Asparaginsäure mit hydrierbaren Schutzgruppen am N- und C-Terminus,
b) Verestern der Seitenketten-Carbonsäure mit dem tertiären Alkohol gemäß Formel III
HO-CR₁R₂R₃ Formel III
wobei R₁, R₂, R₃ unabhängig voneinander eine lineare Kohlenwasserstoffkette mit einer Länge von zwei bis sechs Kohlenstoffatomen darstellen,
c) Hydrogenolyse der hydrierbaren Schutzgruppen am N- und C-Terminus,
d) Einführen einer Fluorenylmethoxycarbonyl- oder einer Benzyloxycarbonylgruppe am N-Terminus.

5. Verfahren nach Anspruch 4, **dadurch gekennzeichnet, dass** die Veresterung des Schrittes b) mit einem Carbodiimid durchgeführt wird.

6. Verfahren für die Peptidsynthese durch
a) Bereitstellen einer Aminosäure oder eines Peptids, die/das über ihren/seinen C-Terminus an eine Festphase gebunden ist,
b) Umsetzen der festphasengebundenen Aminosäure oder des festphasengebundenen Peptids des Schrittes a) mit einer Verbindung der Formel la und/oder Ib wie in Anspruch 1 definiert, in der R₄ 9-Fluorenylmethoxycarbonyl (Fmoc) bedeutet, in der die Verbindung der Formel la und/oder Ib mit dem N-Terminus verknüpft ist,
c) Abspalten der Fmoc-Gruppe durch Alkalibehandlung.

7. Verfahren nach Anspruch 6, **dadurch gekennzeichnet, dass** die Alkalibehandlung des Schrittes c) mit einem oder mehreren sekundären Aminen durchgeführt wird.

8. Verfahren nach Anspruch 6 oder 7, **dadurch gekennzeichnet, dass** die Alkalibehandlung des Schrittes c) mit einem Gemisch enthaltend ein oder mehrere sekundären Amine und eine organische Säure und/oder einen sauren Alkohol durchgeführt wird.

9. Verfahren nach einem oder mehreren der Ansprüche 6 bis 8, **dadurch gekennzeichnet, dass** die Reaktion in Schritt b) in Gegenwart von HBTU, TBTU, HATU, TATU, HCTU, TCTU oder PyBOP durchgeführt wird.

## Revendications

1. Composés de formule la et/ou Ib 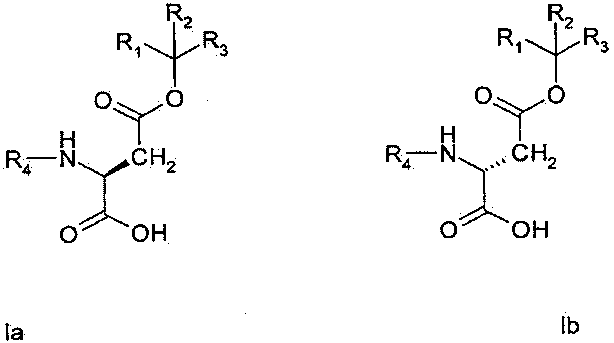 dans lesquels
R₁, R₂, R₃ représentent, indépendamment les uns des autres, une chaîne hydrocarbonée linéaire d'une longueur allant de deux à six atomes de carbone,
R₄ représente benzyloxycarbonyle, 9-fluorénylméthoxycarbonyle ou H.

2. Composé selon la revendication 1, **caractérisé en ce que** R₁, R₂, R₃ possèdent tous le même nombre d'atomes de carbone.

3. Composé selon la revendication 1 ou 2, **caractérisé en ce que** R₁, R₂, R₃ sont -CH₂-CH₃ ou -CH₂-CH₂-CH₃.

4. Méthode de préparation du composé de formule la et/ou Ib, par
a) la fourniture d'un acide aspartique ayant des groupements protecteurs hydrogénisables au niveau de l'extrémité N- et C-terminale,
b) l'estérification de l'acide carboxylique de la chaîne latérale par l'alcool tertiaire selon la formule III
HO-CR₁R₂R₃ formule III
où R₁, R₂, R₃ représentent, indépendamment les uns des autres, une chaîne hydrocarbonée linéaire d'une longueur allant de deux à six atomes de carbone,
c) l'hydrogénolyse des groupements protecteurs hydrogénisables au niveau de l'extrémité N- et C-terminale,
d) l'introduction d'un groupement fluorénylméthoxycarbonyle ou benzyloxycarbonyle au niveau de l'extrémité N-terminale.

5. Méthode selon la revendication 4, **caractérisée en ce que** l'estérification de l'étape b) est effectuée avec un carbodiimide.

6. Méthode de synthèse de peptides, par
a) la fourniture d'un acide aminé ou d'un peptide lié à une phase solide via son extrémité C-terminale,
b) la réaction de l'acide aminé ou du peptide lié à une phase solide de l'étape a) avec un composé de formule la et/ou Ib tel que défini selon la revendication 1, où R₄ est 9-fluorénylméthoxycarbonyle (Fmoc) ce par quoi le composé de formule la et/ou Ib est fixé à l'extrémité N-terminale,
c) le clivage du groupement Fmoc par un traitement alcalin.

7. Méthode selon la revendication 6, **caractérisée en ce que** le traitement alcalin de l'étape c) est effectué avec une ou plusieurs amines secondaires.

8. Méthode selon la revendication 6 ou 7, **caractérisée en ce que** le traitement alcalin de l'étape c) est effectué avec un mélange comprenant une ou plusieurs amines secondaires et un acide organique et/ou un alcool acide.

9. Méthode selon l'une ou plusieurs parmi les revendications 6 à 8, **caractérisée en ce que** la réaction dans l'étape b) est effectuée en présence de HBTU, TBTU, HATU, TATU, HCTU, TCTU ou PyBOP.
